# EUROPEAN PATENT APPLICATION

(11) **EP 4 270 010 A1**
(43) Date of publication of application: **01.11.2023**
(21) Application number: 21915333.5
(22) Date of filing: 28.12.2021
(51) Int. Cl.: G01N 33/86

(54) **METHOD FOR EVALUATING BLOOD COAGULATION PERFORMANCE, AND METHOD FOR INSPECTING RISK OF THROMBOSIS**

(30) Priority: 28.12.2020 JP 2020219401
(71) Applicant: Fujimori Kogyo Co., Ltd., Tokyo 112-0002 (JP)
(72) Inventor: HOSOKAWA, Kazuya, Tokyo 112-0002 (JP)
(74) Representative: Jones, Nicholas Andrew
(86) International application number: PCT/JP2021/048977
(87) International publication number: WO 2022/145475

(57) **Abstract**

A method of evaluating extrinsic blood coagulation ability in vitro, characterized in that a contact factor inhibitor and an inhibitor for a tissue factor pathway inhibitor (TFPI) are added to a blood sample to evaluate blood coagulation ability, and a method of evaluating intrinsic blood coagulation ability in vitro, characterized in that both the contact factor inhibitor and an extrinsic blood coagulation inhibitor are added to a blood sample to evaluate blood coagulation ability.

## Description

### [Technical Field]

The present invention relates to a method of evaluating blood coagulation ability and a method of determining a risk of thrombosis.

### [Background Art]

Blood coagulation functions have been known to be enhanced in cancer, diabetes, various infectious and chronic inflammatory diseases, increasing a risk of thrombosis. In particular, thrombosis in cancer patients (cancer-related thrombosis) occupies the second most common cause of death among cancer patients, resulting in serious problems, however, there has been no simple and appropriate test method of evaluating a hypercoagulable condition of blood coagulation.

Thrombogenicity due to cancer, diabetes, and infectious diseases is related to micro particles derived from vascular endothelial cells and platelets, acid phospholipids on a surface of activated platelets, tissue factors in blood, and the like, and in particular cancer-related thrombosis development is considered to have one of the major causes thereof, which is activation of extrinsic coagulation by expression of tissue factors in monocytes and vascular endothelial cells, due to an immune response to tissue factors contained on a surface of tumor cells and in the tumor cells, and the tumor cells (Non-Patent Literature 1).

Therefore, an inspection of the risk of thrombosis development in these diseases should comprehensively analyze the aforementioned factors.

Blood coagulation tests, such as APTT (activated partial thromboplastin time), PT (prothrombin time), ROTEM (trademark: rotational thromboelastometry), and TEG (thromboelastography) are, on the other hand, used for the purpose of mainly evaluating deletion of a blood coagulation factor of hemophilia and the like, and a hemostatic function (hemorrhagic tendency) in the perioperative period, and not used to evaluate an extent of shortening of clotting time caused by thrombogenicity due to cancer or other causes.

### [Citation List]

### [Non-Patent Literature]

[Non-Patent Literature 1]: The Japanese Society on Thrombosis and Hemostasis Website Glossary "Cancer and Coagulation" (https://www.jsth.org/glossary_detail/?id=291)

### [Summary of Invention]

### [Problem to be solved by the invention]

As described above, an appropriate method of evaluating blood hypercoagulation caused by various factors, has been demanded.

An object of the present invention is to provide a method of appropriately evaluating blood hypercoagulation pathology caused by extrinsic coagulation, micro particles, platelet activation, and the like, in a blood coagulation test.

### [Means to solve the problem]

The present inventor intensively studied in order to solve the aforementioned problem. As a result, the present inventors have found that blood hypercoagulable conditions related to cancer, diabetes, and infectious diseases can easily be evaluated by adding a contact factor inhibitor (one or more of an FXI inhibitor, an FXII inhibitor, a kallikrein inhibitor, and the like) and an inhibitor for a Tissue Factor Pathway Inhibitor (TFPI) to a blood sample to evaluate blood coagulation ability, and thus have completed the present invention.

The present invention provides a method of evaluating blood coagulation ability in vitro, comprising analyzing blood coagulation ability using a blood sample to which a contact factor inhibitor and an inhibitor for a tissue factor pathway inhibitor (TFPI inhibitor) are added.

The present invention also provides a method of evaluating intrinsic blood coagulation ability in vitro, comprising analyzing blood coagulation ability using a blood sample to which both the contact factor inhibitor and an extrinsic blood coagulation inhibitor are added.

The present invention also provides a reagent for analyzing extrinsic blood coagulation ability in vitro, containing the contact factor inhibitor and the TFPI inhibitor.

The present invention also provides a reagent for analyzing intrinsic blood coagulation ability in vitro, containing the contact factor inhibitor and the extrinsic blood coagulation inhibitor.

Here, the contact factor inhibitor may be an FXI inhibitor, an intrinsic blood coagulation, and/or a kallikrein inhibitor. Analysis may be performed by ROTEM and/or TEG.

Moreover, the blood sample may be a blood sample of a cancer patient or a patient suspected of having cancer.

### [Advantageous Effects of Invention]

According to the method of the present invention, by adding a contact factor inhibitor, (an FXI inhibitor, an FXII inhibitor, and/or a kallikrein inhibitor and the like) and a TFPI inhibitor, the blood coagulation ability can be analyzed, sensitively reflecting blood hypercoagulable conditions caused by trace amounts of tissue factors, micro particles, platelet activation, and the like.

Furthermore, whether the blood hypercoagulable condition is due to a trace amount of a tissue factor in blood or other factors (for example, microparticles and acid phospholipids such as activated platelets) can be analyzed by comparing a blood clotting time when having added a contact factor inhibitor and a TFPI inhibitor with that when having added extrinsic blood coagulation inhibitors (FVII inhibitor, tissue factor inhibitor, and the like).

Both a contact factor inhibitor and an extrinsic blood coagulation inhibitor are added to a blood sample to analyze blood coagulation ability, thereby enabling an evaluation of an intrinsic blood hypercoagulable condition caused by micro particles, acid phospholipids such as activated platelets, and the like while an influence of tissue factors in blood is excluded.

### [Brief description of Drawings]

[Figure 1] A view illustrating the results of Example 1.
[Figure 2] A view illustrating the results of Example 2.
[Figure 3] A view illustrating the results of Example 3.

### [Description of Embodiments]

The method of analyzing blood coagulation of the present invention comprises analyzing blood coagulation ability using a blood sample to which a contact factor inhibitor and an inhibitor for a tissue factor pathway inhibitor (TFPI) are added.

A blood sample is preferably a whole blood sample, and it may be a blood sample that is subjected to anticoagulation treatment with citric acid or the like. In such a case, the anticoagulation treatment can be released with calcium or the like to initiate a blood coagulation reaction, as described below.

The blood sample is preferably a blood sample from a patient with a disease such as cancer or from a patient suspected of having a disease such as cancer.

In the method of the present invention, an FXI (blood coagulation factor 11) inhibitor, an FXII (blood coagulation factor 12) inhibitor, a kallikrein inhibitor, and the like, are used as contact factor inhibitors, and two or more types thereof are preferably added in admixture therewith.

In particular, a combination of the FXII inhibitor and the kallikrein inhibitor enables favorable evaluation of an extent of shortening due to blood hypercoagulation.

Inhibitors of contact factors that are small molecule synthetics, peptides/proteins, and the like can be used.

The FXI inhibitor is not particularly limited as long as it is a substance having FXI inhibitory activity, and BMS-962212 (J Med Chem. 2017 Dec 14; 60 (23): 9703-9723) and an FXI inhibitory aptamer (Sci Rep. 2017 May 18; 7 (1): 2102. Selection and characterization of a DNA aptamer inhibiting coagulation factor XIa), and the like, can be used. The FXI inhibitor, for example, BMS-962212, is preferably added at a final concentration of 100 nM to 100 µM.

The FXII inhibitor is not particularly limited as long as it is a substance having FXII inhibitory activity, and a corn trypsin inhibitor (CTI) and a peptide inhibitor (for example, bicyclic peptide 61 (BP-61) or bicyclic peptide 73 (BP-73); J Med Chem. 2017 Feb 9; 60 (3):1151-1158), and the like, can be used. The FXII inhibitor, for example, CTI, is preferably added at a final concentration of 10 µg/mL to 200 µg/mL.

In the case of BP61 or BP73, the final concentration thereof is preferably 1 to 100 µg/mL.

The kallikrein inhibitor is not particularly limited, and a synthetic kallikrein inhibitor (for example, PKSI-527((CAS No.: 128837-71-8) Abcam plc), aprotinin, and the like are preferred. The PKSI-527 is preferably added at a final concentration of 1 µM to 1 mM. The aprotinin is preferably added at a final concentration of 10 to 1,000 KIU/mL.

More preferably, adding both the FXII inhibitor and the kallikrein inhibitor sufficiently inhibits a blood coagulation reaction caused by contact factor activation in whole blood, thereby allowing identification of extrinsic hypercoagulation, which is preferred.

An inhibitor of the contact factor, which is either an inhibitor of activation of unactivated coagulation factors (FXII, FXI) and an inhibitor of enzymatic activity of activated coagulation factors (FXIIa, FXIa), can be used.

TFPI inhibitors that are small molecule and peptide/protein inhibitors, can be used, and are not particularly limited as long as they can inhibit a TFPI function, and specifically a TFPI inhibitor of a small molecule peptide (for example, compound 3; J Biol Chem. 2014 Jan 17; 289 (3):1732-41) is exemplified. The TFPI inhibitor, which is, for example, Compound 3, is added at a final concentration of 1 µg/mL to 200 µg/mL.
Compound 3, Ac-FQSKpNVHVDGYFERL-Aib-AKL-NH₂.

As other TFPI inhibitor, an RNA aptamer inhibitor also can be used.

Examples of the RNA aptamer inhibitor include BAX499 (J Thromb Haemost. 2012 Aug; 10 (8): 1581-90.). For example, BAX499 is added at a final concentration of 1 nM to 1000 nM.

Further, an anti-TFPI antibody and the like having TFPI inhibitory activity also can be used.

Methods of analyzing blood coagulation are not particularly limited, and apparatuses capable of evaluating blood coagulation in whole blood, such as ROTEM (Rotational Thromboelastometry), TEG (Thromboelastography), ClotPro (Hemonetics Corp.), SONOCLOT (Science Corporation), and ACTAS (Fujimori Kogyo Co., Ltd.: WO2018/043420), are preferably used.

Using whole blood as a sample enables favorable evaluation of blood hypercoagulability due to cell components (leukocytes and erythrocytes).

Whether a blood hypercoagulation condition is present can be analyzed by preliminarily adding the contact factor inhibitor and the TFPI inhibitor to healthy blood and blood of patients with hypercoagulability followed by carrying out a blood coagulation test to calculate their clotting times and coagulation waveforms, and then observing that a clotting time or waveform of a sample is close to which of those of the aforementioned healthy blood or blood of the patients.

Whether the hypercoagulable condition is mainly due to a blood tissue factor in blood or to other factors (for example, micro particles and platelet activation) can also be evaluated by comparing the results of a blood coagulation test carried out by having added the contact factor inhibitor and the TFPI inhibitor to a blood sample, with those in a case in which no TFPI inhibitor was added (in the absence of the TFPI inhibitor), i.e., the results of a blood coagulation test in which only the contact factor inhibitor was added to the blood sample.

For example, it is possible to evaluate a factor responsible for the hypercoagulable condition by comparing blood clotting time and ROTEM/TEG waveforms with and without the TFPI inhibitor and by evaluating the extent of prolongation of the time.

It is also possible to analyze a factor responsible for the hypercoagulable condition by comparing the results of an extrinsic blood coagulation test carried out with the contact factor inhibitor and TFPI inhibitor, with those of an intrinsic blood coagulation test carried out in the presence of extrinsic blood coagulation inhibitors such as an FVII inhibitor (also called an FVIIa inhibitor), a tissue factor inhibitor, and an inactivated FVIIa.

That is, an intrinsic hypercoagulable condition of blood hypercoagulation not caused by a tissue factor in blood can be evaluated by analyzing the blood coagulation ability using a blood sample to which both the contact factor inhibitor and the extrinsic blood coagulation inhibitor are added.

Examples of extrinsic blood coagulation inhibitors used in an intrinsic blood coagulation test include an FVII (blood coagulation factor 7) inhibitor, a tissue factor inhibitor, an inactivated FVIIa, and the like. The FVII inhibitor that is an inhibitor for activation of FVII or an inhibitor for enzymatic activity of FVIIa, can be used. For example, as the FVII inhibitors, a synthetic peptide (A-183X; J Biol Chem. 2003 Jun 13; 278 (24): 21823-30) and a synthetic small molecule (PCI-27483; 2019; 96 (4): 217-222. doi: 10.1159/000495988. Epub 2019 Mar 7.), an inactivated FVIIa (Eur JVasc Endovasc Sur. 1998 Jun; 15 (6): 515-20. doi: 10.1016/s1078-5884 (98) 80112-3.), antibodies for tissue factors, and the like, can be used.

As the FVII inhibitor, for example, the A183-X can be added at a final concentration of 1 nM to 1 µM.

Alternatively, the PCI-27483 can be added at a final concentration of 10 nM to 10 µM.

Alternatively, the inactivated FVIIa can be added at a final concentration of 10 ng/mL to 100 µg/mL.

Whole blood is preferably used for an analysis of blood coagulation performed by addition of an extrinsic blood coagulation inhibitor. When blood that underwent anticoagulation treatment with sodium citrate is used, an analysis of blood coagulation can be performed by having added the extrinsic blood coagulation inhibitor and calcium chloride.

With regard to an analysis of intrinsic blood coagulation in which extrinsic blood coagulation was inhibited, contact factor inhibitor (FXI inhibitor, FXII inhibitor, kallikrein inhibitor) is preferably added together with an extrinsic blood coagulation inhibitor (inhibitor of FVII or tissue factors).

As the contact factor inhibitors, the FXI (blood coagulation factor 11) inhibitor, the FXII (blood coagulation factor 12) inhibitor, and the kallikrein inhibitor, described above, and the like, are used, and their preferred concentration ranges are also the same as those described above.

When both the extrinsic blood coagulation inhibitors and contact factor inhibitors are added, a trace amount of blood coagulation activator may be further added as an activating reagent for intrinsic blood coagulation. Trace amounts of blood coagulation activators include FX activators such as an activated coagulation factor (FXa and thrombin) and FX (blood coagulation factor 10) activating enzyme derived from Russell's viper venom (RVV) (RVV-X; J Biol Chem. 1994 Apr 8; 269 (14):10644-50.), and prothrombin activators such as ecarin (Am J Hematol. 2020 Jul;95 (7):863-869.).

In the case of RVV-X, it can be used at a final concentration of 0.1 ng/mL to 1000 ng/mL.

In the case of ecarin, it can be used at a final concentration of 0.1 mU to 100 mU.

These blood coagulation activators are not inhibited by the contact factor inhibitor or the extrinsic coagulation inhibitor.

Moreover, blood coagulation activator is preferably added such that a blood clotting time is 20 to 60 minutes in blood of healthy subjects. The blood clotting time in healthy subjects being 20 minutes or longer enables detection of shortening of blood clotting time in blood of a patient with hypercoagulability.

Alternatively, the extrinsic blood coagulation inhibitor and kallikrein inhibitor may be used in combination.

In the case of using the kallikrein inhibitor singly as the contact factor inhibitor in addition to the extrinsic blood coagulation inhibitor, FXII is not directly inhibited, but the mutual activation of FXIIa and kallikrein is inhibited, thereby inhibiting FXIIa production, which therefore prolongs activation in healthy subjects to 20 minutes or longer and enables detection of tendency where a clotting time is shortened in a patient with hypercoagulability.

Furthermore, by comparing with the results without the TFPI inhibitor, or comparing with the analysis results of intrinsic blood coagulation in the presence of the extrinsic blood coagulation inhibitor to analyze a cause of hypercoagulation, it is possible to reflect the analysis result in treatment, such as administration of an anticoagulant drug if the hypercoagulation is derived from a tissue factor or of an antiplatelet drug if it is due to platelet activation.

For example, in thrombosis related to cancer, various factors of hypercoagulation conditions are expressed depending on types of tumors and therapeutic agents.

For example, tumor cells may directly express a tissue factor or release microparticles that expressed a tissue factor. Moreover, certain types of tumor cells have expressed a glycoprotein called podoplanin, which directly activates platelets. Acid phospholipids on a surface of activated platelets promote blood coagulation. The tumor cells release microparticles derived from the vascular endothelium due to vascular endothelial cell damages caused by treatment with various anticancer drugs. In particular, development of venous thrombosis due to the vascular endothelial cell damages caused by angiogenesis inhibition (VEGF inhibitor drug) has been reported in many cases.

Therefore, there are a plurality of factors that contribute to tumor-related hypercoagulation pathology, and with a strong influence of tissue factor, a difference in blood coagulation in the presence and absence of the TFPI inhibitor or a difference between the analysis results of the extrinsic coagulation and those of the intrinsic coagulation, described above, becomes large, on the other hand, with strong influences of micro particles and platelet activation, the difference becomes small.

In such a way, by comparing blood coagulation in the presence and absence of the inhibitors for TFPI, or by comparing the extrinsic and intrinsic blood coagulation, the degree and factors of blood hypercoagulation can be analyzed.

Blood samples may be analyzed for blood coagulation by adding the contact factor inhibitor and the TFPI inhibitor immediately after collection of the blood, however, it may be difficult to carry out a test for blood coagulation immediately after blood collection.

Therefore, it is preferable to collect blood in a blood collection container containing a reversible anticoagulant, then add a reagent releasing the anticoagulation treatment of the anticoagulant contained in the blood collection container, the contact factor inhibitor, and the TFPI inhibitor, at the start of the analysis, followed by analyzing blood coagulation.

For example, in a case in which blood is collected in a container containing sodium citrate as a reversible anticoagulant, calcium chloride, the contact factor inhibitor, and the TFPI inhibitor are added to start analyzing blood coagulation.

Such combinations of the reversible anticoagulant and the release agent include heparin and a heparin neutralizer (heparinase, polybrene, protamine, and the like), a thrombin inhibitor DNA aptamer and an antisense DNA for the thrombin inhibitor DNA aptamer.

When citric acid is used as the reversible anticoagulant, it is convenient to be able to use commercially available blood collection tubes (or blood collection containers) containing sodium citrate.

However, using a vacuum blood collection tube containing sodium citrate with a rubber stopper made of butyl rubber allows blood to contact the rubber stopper and activate blood coagulation, which may shorten a clotting time. Therefore, a blood collection tube containing citric acid without a rubber stopper is preferably used. For example, a Benoject blood collection tube sealed with a laminate film (Terumo Corporation) can be used as a blood collection tube without a rubber stopper.

When analyzing blood collected by using a blood collection tube containing citric acid with a rubber stopper, in addition to calcium chloride and the contact factor inhibitor and the TFPI inhibitor, small amounts of anticoagulant substances such as heparin-like substances (heparin, small molecule heparin, pentasaccharide, heparan sulfate) may be added to blood upon analyzing blood coagulation in order to prolong a clotting time (WO2017/119508).

In this case, a blood clotting time in healthy subjects by using an apparatus such as ROTEM or ClotPro (Enicor GmbH) is preferably adjusted to approximately 20 to 60 minutes.

Alternatively, when using a blood collection tube containing sodium citrate with a rubber stopper, it is possible to inhibit activation of a contact factor due to the rubber stopper by using a blood collection tube containing sodium citrate and to which an FXII inhibitor is preliminarily added.

When whole blood underwent anticoagulation treatment with sodium citrate, functions of cell components such as platelets and leukocytes may be reduced. Therefore, in a case in which activation conditions of the platelets and leukocytes are to be reflected, combinations of the reversible anticoagulant and the release agent other than sodium citrate and calcium chloride, e.g., heparin and heparinase, thrombin inhibitory DNA aptamer and antisense DNA are preferably employed.

The present invention also provides a reagent kit for analyzing extrinsic blood coagulation ability in vitro, containing a contact factor inhibitor and a TFPI inhibitor. The types of contact factor inhibitor and TFPI inhibitor are as described above, and they are diluted upon their use and added at final concentrations suitable for analyzing blood coagulation ability (the preferred ranges of final concentrations are as described above).

The present invention also provides a reagent kit for analyzing intrinsic blood coagulation ability in vitro, containing the contact factor inhibitor and the extrinsic blood coagulation inhibitor. The types of contact factor inhibitor and extrinsic blood coagulation inhibitor are as described above, and they are diluted upon their use and added at final concentrations suitable for analyzing blood coagulation ability (the preferred ranges of final concentrations are as described above).

### [Examples]

The present invention will be more specifically described below by referring to Examples, but the present invention is not limited to the embodiments below. The blood samples used in the Examples were collected from healthy humans unless otherwise described.

### Example 1

ROTEM (Instrumentation Laboratory Co (IL)) was used to analyze blood coagulation.

Blood was collected using a blood collection tube (5 mL) containing 3.2% sodium citrate manufactured by Terumo Corporation.

This blood collection tube was sealed with a laminate film, and no rubber stopper is used.

To each of 300 µL of whole blood were added the following reagents to analyze blood coagulation waveforms.
(1) 20 µL of a Startem reagent (IL) that is a calcium chloride reagent (final concentration 12 mM)
(2) 20 µL of a Startem reagent (IL) (final concentration 12 mM) and a TFPI inhibitor (compound 3) (final concentration 100 µg/mL)
(3) 20 µL of a Startem reagent (IL) (final concentration 12 mM), a TFPI inhibitor (compound 3) (final concentration 100 µg/mL), and a rabbit brain-derived tissue thromboplastin (final concentration 0.45 ng/mL)
(4) 20 µL of a Startem reagent (IL) (final concentration 12 mM), a TFPI inhibitor (compound 3) (final concentration 100 µg/mL), and a rabbit brain-derived tissue thromboplastin (final concentration 0.9 ng/mL)

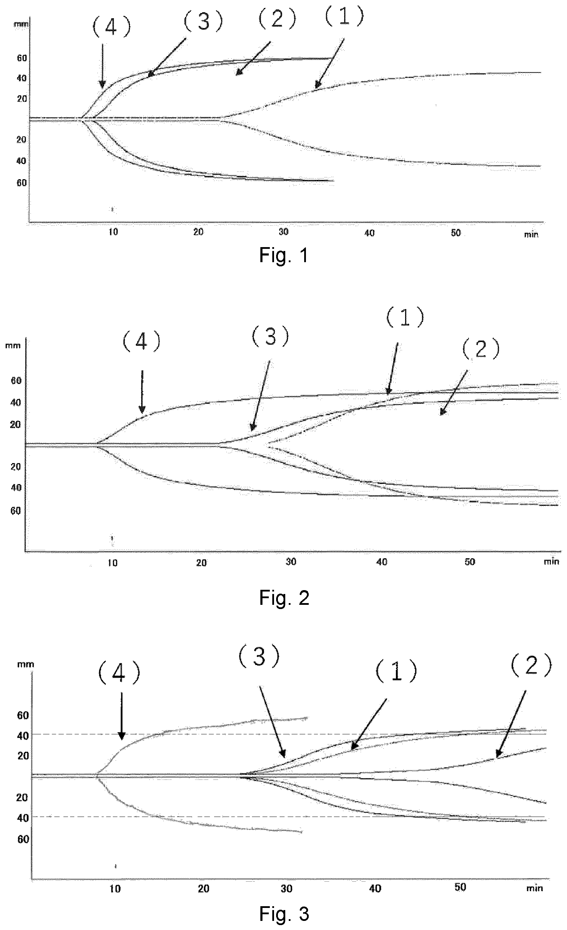

The results are shown in Figure 1. The clotting time (CT) of (1) with only calcium chloride was 1336 seconds, whereas the CT of (2) with calcium chloride and the inhibitor for TFPI was shortened to 801 seconds. Furthermore, in (3) and (4), the CTs were shortened to 452 and 371 seconds by the addition of the trace amount of tissue thromboplastin.

The blood coagulation-promoting effect by the TFPI inhibitor in the presence of the trace amount of tissue thromboplastin was demonstrated.

### Example 2

ROTEM (IL) was used to analyze blood coagulation.

Blood was collected using a blood collection tube (5 mL) containing 3.2% sodium citrate manufactured by Terumo Corporation.

This blood collection tube was sealed a laminated film, and no rubber stopper is used.

To each of 300 µL of whole blood were added the following reagents to analyze blood coagulation waveforms.
(1) Calcium chloride (final concentration 12 mM) and CTI (final concentration 50 µg/mL)
(2) Calcium chloride (final concentration 12 mM), CTI (final concentration 20 µg/mL), and PKSI-527 (final concentration 40 µM)
(3) Calcium chloride (final concentration 12 mM), CTI (final concentration 20 µg/mL), PKSI-527 (final concentration 40 µM), and a rabbit brain-derived tissue thromboplastin (final concentration 0.45ng/mL)
(4) Calcium chloride (final concentration 12 mM), CTI (final concentration 20 µg/mL), PKSI-527 (final concentration 40 µM), a rabbit brain-derived tissue thromboplastin (final concentration 0.45 ng/mL), and a TFPI inhibitor (compound 3) (final concentration 100 µg/mL)

The results are shown in Figure 2. The addition of both the FXII factor inhibitor and kallikrein inhibitor prolonged the clotting time (CT) of (2) to 2245 seconds. Furthermore, the CT of (3) with the addition of the trace amount of tissue thromboplastin was shortened to 1304 seconds and the CT of (4) with the addition of the trace amount of tissue thromboplastin and the TFPI inhibitor was shortened to 489 seconds. The addition of the FXII inhibitor and kallikrein inhibitor prolonged the clotting time upon the addition of no trace amount of tissue thromboplastin prolonged the clotting time, and further the addition of the trace amount of tissue thromboplastin and the TFPI inhibitor significantly prolonged the clotting time.

That is, the presence of the FXII inhibitor, kallikrein inhibitor and TFPI inhibitor allowed for evaluation of the promoting effect of the trace amount of tissue thromboplastin on thrombus formation.

The blood coagulation in the absence of the trace amount of tissue factor is delayed in Example 2 with the addition of the contact factor inhibitor and the kallikrein inhibitor, as compared with that in Example 1, however, the blood clotting time is greatly shortened when the trace amount of tissue thromboplastin and the TFPI inhibitor were added. In other words, the addition of the contact factor inhibitor and TFPI inhibitor enables evaluation of likelihood of thrombosis by the trace amount of tissue thromboplastin.

### Example 3

ROTEM (IL) was used to analyze blood coagulation.

Blood was collected using a blood collection tube (2 mL) containing 3.2% sodium citrate manufactured by Becton, Dickinson and Company. A rubber stopper is used for this blood collection tube.

To each whole blood were added the following reagents to analyze blood coagulation waveforms.
(1) Calcium chloride (final concentration 12 mM) and CTI (final concentration 50 µg/mL)
(2) Calcium chloride (final concentration 12 mM), CTI (final concentration 20 µg/mL), PKSI-527 (final concentration 40 µM), and heparan sulfate (final concentration 1 µg/mL)
(3) Calcium chloride (final concentration 12 mM), CTI (final concentration 20 µg/mL), PKSI-527 (final concentration 40 µM), heparan sulfate (final concentration 1 µg/mL), and a rabbit brain-derived tissue thromboplastin (final concentration 0.9 ng/mL)
(4) Calcium chloride (final concentration 12 mM), CTI (final concentration 20 µg/mL), PKSI-527 (final concentration 40 µM), heparan sulfate (final concentration 1 µg/mL), a rabbit brain-derived tissue thromboplastin (final concentration 0.9 ng/mL), and a TFPI inhibitor (compounds) (final concentration 100 µg /mL).

The results are shown in Figure 3. In the case of having used the blood collection tube with a rubber stopper, the rubber stopper may influence the blood, accelerating blood coagulation. As shown in (2), the coagulation-promoting effect of the rubber stopper can be inhibited by heparan sulfate.

Furthermore, the CT of (3) with the trace amount of tissue thromboplastin was 1451 seconds, while the CT of (4) with the TFPI inhibitor was further shortened to 440 seconds.

The addition of the contact factor inhibitor, heparan sulfate, and TFPI inhibitor made it possible to evaluate the blood coagulation-promoting effect of the trace amount of tissue factor.

### Example 4

ROTEM (IL) was used to analyze blood coagulation.

Blood was collected using a blood collection tube containing 3.2% sodium citrate manufactured by Terumo Corporation.

This blood collection tube was sealed with a laminate film, and no rubber stopper is used.

To each of 300 µL of whole blood were added the following reagents to analyze blood coagulation waveforms.
(1) Calcium chloride (final concentration 12 mM) and CTI (final concentration 20 µg/mL)
(2) Calcium chloride (final concentration 12 mM) and PKSI-527 (final concentration 40 µM)
(3) Calcium chloride (final concentration 12 mM), CTI (final concentration 20 µg/mL), and PKSI-527 (final concentration 40 µM)
(4) A Startem reagent (IL) (20 µL)
(5) Calcium chloride (final concentration 12 mM), CTI (final concentration 20 µg/mL), and a human recombinant tissue factor (Dade Innovin; Siemens AG) (1% of 1,000-fold dilution was added.)
(6) Calcium chloride (final concentration 12 mM), PKSI-527 (final concentration 40 µM), and a human recombinant tissue factor (Dade Innovin; Siemens AG) (1% of 1,000-fold dilution was added.)
(7) Calcium chloride (final concentration 12 mM), CTI (final concentration 20 µg/mL), PKSI-527 (final concentration 40 µM), and a human recombinant tissue factor (Dade Innovin; Siemens AG) (1% of 1,000-fold dilution was added.)
(8) A Startem reagent (IL) and a human recombinant tissue factor (Dade Innovin; Siemens AG) (1% of 1,000-fold dilution was added.)

The results of CT and CFT are shown in Table 1.

**[Table 1]**

| | (1) | (2) | (3) | (4) | (5) | (6) | (7) | (8) |
|---|---|---|---|---|---|---|---|---|
| CT | 1, 187 | 1,035 | 1,532 | 634 | 592 | 602 | 623 | 552 |
| CFT | 399 | 259 | 291 | 222 | 213 | 216 | 227 | 216 |

As compared to the addition of the FXII factor inhibitor singly in (1) and that of the kallikrein inhibitor singly in (2), the addition of both in (3), increased the extent of prolongation of clotting time (CT).

On the other hand, the clotting times of (5), (6), and (7) with the addition of the trace amount of Dade Innovin resulted in around 600 seconds of CT values, similar values among them. Therefore, the addition of both FXII inhibitor and kallikrein inhibitor increases the extent of shortening of clotting times when the trace amount of tissue factor was added, and hypercoagulation can be easily detected. The extents of shortening of clotting times without the addition of contact factor inhibitors in (4) and (8) were further smaller, indicating that both cases were not suitable for evaluation of hypercoagulation.

### Example 5

ROTEM (IL) was used to analyze blood coagulation.

Blood was collected using a blood collection tube (5 mL) containing 3.2% sodium citrate manufactured by Terumo Corporation.

This blood collection tube was sealed with a laminate film, and no rubber stopper is used.

To each of 300 µL of whole blood were added the following reagents to analyze blood coagulation waveforms.
(1) A Startem reagent (IL) (20 µL)
(2) Calcium chloride (final concentration 12 mM), CTI (final concentration 20 µg/mL), and PKSI-527 (final concentration 40 µM)
(3) Calcium chloride (final concentration 12 mM), CTI (final concentration 20 µg/mL), PKSI-527 (final concentration 40 µM), and a TFPI inhibitor (compounds) (final concentration 50 µM)
(4) A Startem reagent (IL) (20 µL) and a human umbilical vein endothelial cell-derived exosome (final concentration 1 µg/mL)
(5) Calcium chloride (final concentration 12 mM), CTI (final concentration 20 µg/mL), PKSI-527 (final concentration 40 µM), and a human umbilical vein endothelial cell (HUVEC)-derived exosome (final concentration 1 µg/mL)
(6) Calcium chloride (final concentration 12 mM), CTI (final concentration 20 µg/mL), PKSI-527 (final concentration 40 µM), TFPI inhibitor (compound 3) (final concentration 50 µM), and a human umbilical vein endothelial cell (HUVEC)-derived exosome (final concentration 1 µg/mL)
(7) A Startem reagent (IL) (20 µL) and a human malignant melanoma A375 cell-derived exosome (final concentration 1 µg/mL)
(8) Calcium chloride (final concentration 12 mM), CTI (final concentration 20 µg/mL), PKSI-527 (final concentration 40 µM), and a human malignant melanoma A375 cell-derived exosome (final concentration 1 µg/mL)
(9) Calcium chloride (final concentration 12 mM), CTI (final concentration 20 µg/mL), PKSI-527 (final concentration 40 µM), TFPI inhibitor (compounds) (final concentration 50 µM), and a human malignant melanoma A375 cell-derived exosome (final concentration 1 µg/mL)
(10) A Startem reagent (IL) (20 µL) and a human histone H4 (manufactured be Abcam plc) (final concentration 2 µM)
(11) Calcium chloride (final concentration 12 mM), CTI (final concentration 20 µg/mL), PKSI-527 (final concentration 40 µM), and a human histone H4 (manufactured be Abcam plc) (final concentration 2 µM)
(12) Calcium chloride (final concentration 12 mM), CTI (final concentration 20 µg/mL), PKSI-527 (final concentration 40 µM), TFPI inhibitor (compounds) (final concentration 50 µg/mL), and a human histone H4 (manufactured be Abcam plc) (final concentration 2 µM)

The results of CT values are shown in Table 2.

**[Table 2]**

| | (1) | (2) | (3) | (4) | (5) | (6) | (7) | (8) | (9) | (10) | (11) | (12) |
|---|---|---|---|---|---|---|---|---|---|---|---|---|
| CT | 1, 032 | 2, 769 | 1, 558 | 1,027 | 2143 | 1, 321 | 593 | 710 | 488 | 806 | 1791 | 905 |
| CFT | 353 | 311 | 351 | 253 | 370 | 227 | 228 | 371 | 233 | 171 | 232 | 144 |

The exosomes derived from vascular endothelial cells and cancer cells are known to promote blood coagulation by having tissue factors and acid phospholipids on their surfaces, which can be a cause of thrombosis.

The histone is also known to promote thrombus formation by activating platelets.

The addition of the CTIs and kallikrein inhibitors in (2), (5), (8), and (11) resulted in a greater extent of shortening of blood coagulation with the addition of the thrombus-promoting substances, as compared to that of STARTEM reagents in (1), (4), (7), and (10), and in particular the addition of the A375 cell-derived exosome in (8) greatly shortened the blood clotting time.

Adding the CTI, kallikrein inhibitor and TFPI inhibitor shortened the blood clotting time both with the addition of the A375 cell-derived exosome and the histone H4 in (9) and (12).

Furthermore, the degree of hypercoagulation was found to be greater in the exosome derived from cancer cells (A375) than in the exosome derived from normal cells (HUVEC).

### Example 6

ROTEM (IL) was used to analyze blood coagulation.

Blood was collected using a blood collection tube (5 mL) containing 3.2% sodium citrate manufactured by Terumo Corporation.

This blood collection tube was sealed with a laminate film, and no rubber stopper is used.

To each of 300 µL of whole blood was added a lipopolysaccharide derived from Escherichia coli (LPS; manufactured by Merk & Co.) (final concentration 1 µg/mL) or phorbol myristate acetate (PMA; manufactured by Merk & Co.) (final concentration 50 nM) or a human histone H4 (manufactured by Abcam plc) (final concentration 2 µM), each mixture was incubated at 37°C for 4 hours, and then further added with each of reagents (a) to (c) to analyze blood coagulation waveforms.
(a) A Startem reagent (IL) (20 µL)
(b) Calcium chloride (final concentration 12 mM), CTI (final concentration 20 µg/mL), and PKSI-527 (final concentration 40 µM)
(c) Calcium chloride (final concentration 12 mM), CTI (final concentration 20 µg/mL), PKSI-527 (final concentration 40 µM), and a TFPI inhibitor (compound 3) (final concentration 50 µM)

The final combinations of reagents to be added will be described below.

After the incubation at 37°C for 4 hours without addition of reagents to blood, (a), (b), or (c) was added to analyze blood coagulation:
(1) A Startem reagent (IL) (20 µL)
(2) Calcium chloride (final concentration 12 mM), CTI (final concentration 20 µg/mL) and PKSI-527 (final concentration 40 µM)
(3) Calcium chloride (final concentration 12 mM), CTI (final concentration 20 µg/mL), PKSI-527 (final concentration 40 µM), and a TFPI inhibitor (compound 3) (final concentration 50 µM)

Following incubation at 37°C for 4 hours with addition of LPS to blood, (a), (b), or (c) was added to analyze blood coagulation:
(4) A Startem reagent (IL) (20 µL) and an E. coli-derived lipopolysaccharide (LPS; manufactured by Merk & Co.) (final concentration 1 µg/mL)
(5) Calcium chloride (final concentration 12 mM), CTI (final concentration 20 µg/mL), PKSI-527 (final concentration 40 µM), and an E. coli-derived lipopolysaccharide (LPS; manufactured by Merk & Co.) (final concentration (1 µg/mL)
(6) Calcium chloride (final concentration 12 mM), CTI (final concentration 20 µg/mL), PKSI-527 (final concentration 40 µM), TFPI inhibitor (compound 3) (final concentration 50 µM) and an E. coli-derived lipopolysaccharide (LPS; manufactured by Merk &Co.) (final concentration 1 µg/mL)

Following incubation at 37°C for 4 hours with addition of PMAto blood, (a), (b), or (c) was added to analyze blood coagulation:
(7) A Startem reagent (IL) (20 µL) and phorbol myristate acetate (PMA; manufactured by Merk & Co.) (final concentration 50 nM)
(8) Calcium chloride (final concentration 12 mM), CTI (final concentration 20 µg/mL), PKSI-527 (final concentration 40 µM), a human malignant melanoma A375 cell-derived exosome (final concentration 1 µg/mL), phorbol myristate acetate (PMA; manufactured by Merk & Co.) (final concentration 50 nM)
(9) Calcium chloride (final concentration 12 mM), CTI (final concentration 20 µg/mL), PKSI-527 (final concentration 40 µM), a TFPI inhibitor (compound 3) (final concentration 50 µM), a human malignant melanoma A375 cell-derived exosome (final concentration 1 µg/mL), and phorbol myristate acetate (PMA; manufactured by Merk &Co.) (final concentration 50 nM)

Following incubation at 37°C for 4 hours with addition of H4 to blood, (a), (b), or (c) was added to analyze blood coagulation:
(10) A Startem reagent (IL) (20 µL) and a human histone H4 (manufactured by Abcam plc) (final concentration 2 µM)
(11) Calcium chloride (final concentration 12 mM), CTI (final concentration 20 µg/mL), PKSI-527 (final concentration 40 µM), and a human histone H4 (manufactured by Abcam plc) (final concentration 2 µM)
(12) Calcium chloride (final concentration 12 mM), CTI (final concentration 20 µg/mL), PKSI-527 (final concentration 40 µM), a TFPI inhibitor (compound 3) (final concentration 50 µM), and a human histone H4 (manufactured by Abcam plc) (final concentration 2 µM)

The results of CT and CFT are shown in Table 3.

**[Table 3]**

| | (1) | (2) | (3) | (4) | (5) | (6) | (7) | (8) | (9) | (10) | (11) | (12) |
|---|---|---|---|---|---|---|---|---|---|---|---|---|
| CT | 1, 054 | 2, 130 | 1, 435 | 698 | 1, 150 | 603 | 746 | 930 | 700 | 762 | 980 | 663 |
| CFT | 342 | 467 | 296 | 278 | 268 | 315 | 375 | 340 | 261 | 198 | 167 | 119 |

The LPS is known to express a tissue factor on a surface of monocytes via a Toll-like receptor 4 to enhance and renew blood coagulation. The PMA is also known to act on neutrophils and promote thrombus formation by inducing neutrophil extracellular traps (NETs).

With STARTEM reagents in (1), (4), (7), and (10), the extents of shortening due to thrombus-promoting substances such as LPS, PMA, and histone are small. As compared thereto, when the CTI and kallikrein inhibitor were added in (2), (5), (8), and (11), on the other hand, the extents of shortening of blood coagulation due to the addition of thrombus-promoting substances such as LPS, PMA, are large. It was found that when the CTI, kallikrein inhibitor and TFPI inhibitor were further added in (3), (6), (9), and (12), the blood clotting time of blood with thrombus-promoting substances were short, and the further addition of LPS, which promoted a tissue factor expression, greatly promoted blood coagulation in (6).

### (Comparative Example)

ROTEM (IL) was used to analyze blood coagulation.

Blood was collected using a blood collection tube (5 mL) containing 3.2% sodium citrate manufactured by Terumo Corporation.

This blood collection tube was sealed with a laminate film, and no rubber stopper is used.

Plasma and platelet-rich plasma were obtained by centrifuging the obtained blood at 3000 rpm (15 minutes) and 2400 rpm (7 minutes). To these plasma samples were added the same reagents (LPS, PMA, and histone) as in Example 6, and each mixture was incubated at 37°C for 4 hours followed by addition of each of the same reagents as in (1) to (12) of Example 6 to analyze blood coagulation waveforms.

The results are shown in Table 4.

**[Table 4]**

| The symbol "=` denotes a case in which no blood coagulation occurs for 60 minutes or longer (no CT and CFT values) | | | | | | | | | | | | | | | | | |
|---|---|---|---|---|---|---|---|---|---|---|---|---|---|---|---|---|---|
| plasma | (1) | (2) | (3) | (4) | | (5) | (6) | | (7) | | (8) | (9) | | (10) | | (11) | (12) |
| CT | 1815 | - | - | 1878 | | - | - | | 2267 | | - | - | | - | | - | - |
| CFT | 1326 | - | - | 1285 | | - | - | | - | | - | - | | - | | - | - |
| | | | | | | | | | | | | | | | | | |

| platelet rich plasma | (1) | (2) | (3) | | (4) | (5) | | (6) | | (7) | (8) | | (9) | | (10) | (11) | (12) |
|---|---|---|---|---|---|---|---|---|---|---|---|---|---|---|---|---|---|
| CT | 1, 059 | - | - | | 1367 | - | | 2613 | | 1077 | - | | 2758 | | 1585 | - | - |
| CFT | 298 | - | - | | 404 | - | | 446 | | 341 | - | | 504 | | 601 | - | - |

When the plasma and platelet-rich plasma were used in the samples, the addition of the contact factor inhibitor often resulted in the blood clotting time of 1 hour or longer (-), which meant that the CT values were not obtained, leading to unsuitable analyses. This indicates that whole blood is preferred for evaluation of hypercoagulation.

### Example 7

ROTEM (IL) was used to analyze blood coagulation.

Blood was collected using a blood collection tube (5 mL) containing 3.2% sodium citrate manufactured by Terumo Corporation.

This blood collection tube was sealed with a laminate film, and no rubber stopper is used.

To each of 300 µL of whole blood were added the following reagents to analyze blood coagulation waveforms.
(1) A Startem reagent (IL) (20 µL)
(2) Calcium chloride (final concentration 12 mM), CTI (final concentration 20 µg/mL), and PKSI-527 (final concentration 40 µM)
(3) Calcium chloride (final concentration 12 mM), CTI (final concentration 20 µg/mL), PKSI-527 (final concentration 40 µM) and an FVIIa inhibitor (A-183X) (final concentration 100 nM)
(4) Calcium chloride (final concentration 12 mM), CTI (final concentration 20 µg/mL), PKSI-527 (final concentration 40 µM), an FVIIa inhibitor (A-183X) (final concentration 100 nM), and Russell's viper venom FX activating enzyme (RVV-FX) (final concentration 5ng/ml)
(5) Calcium chloride (final concentration 12 mM), CTI (final concentration 20 µg/mL), PKSI-527 (final concentration 40 µM), an FVIIa inhibitor (A-183X) (final concentration 100 nM), and ecarin (final concentration 4 mU/ml units)
(6) Calcium chloride (final concentration 12 mM), PKSI-527 (final concentration 40 µM), and an FVIIa inhibitor (A-183X) (final concentration 100 nM)

To the reagents (1) to (6), A375-derived exosomes (final concentration 1 µg/mL) as blood coagulation accelerators were further added to perform a contrast experiment.

The results of CT and CFT are shown in Table 5.

**[Table 5]**

| No addition of A375 derived exosome | | | | | | |
|---|---|---|---|---|---|---|
| | (1) | (2) | (3) | (4) | (5) | (6) |
| CT | 816 | 2, 101 | 2, 666 | 1, 255 | 1331 | 1, 108 |
| CFT | 273 | 549 | - | 339 | 448 | 291 |

| Addition of A375 derived exosome | | | | | | |
|---|---|---|---|---|---|---|
| | (1) | (2) | (3) | (4) | (5) | (6) |
| CT | 521 | 452 | 1, 619 | 1, 074 | 1, 067 | 1, 123 |
| CFT | 245 | 210 | 542 | 193 | 224 | 251 |

In this Example, with the aim of evaluating a hypercoagulable condition of intrinsic blood coagulation in contrast to the evaluation of the hypercoagulable condition of extrinsic blood coagulation (the case (2)), blood coagulation was analyzed for the case of having added CTI, the kallikrein inhibitor, the FVIIa inhibitor, and the trace amount of RVV-FX or ecarin (the case (4) and (5)), and the case of having further added the kallikrein inhibitor and the FVIIa inhibitor (the case (6)).

In (4) and (5), the intrinsic blood coagulation is evaluated by having added the trace amount of RVV (FX activator) or ecarin (prothrombin activator) while the contact factor activation and FVIIa activity were inhibited. The thrombin produced by blood coagulation with the trace amount of ecarin or RVV-FX activates FXI, FVIII, and FV, amplifying the intrinsic blood coagulation.

In (6), with the addition of the FVIIa inhibitor and the kallikrein inhibitor, the blood clotting time due to the activation of intrinsic blood coagulation starting from FIXa produced by contact factor activation in an inhibitory environment of a partial contact factor, is evaluated.

In the absence of the human malignant melanoma A375 cell-derived exosome, the addition of the calcium chloride, CTI, and kallikrein inhibitor in (2) greatly prolongs the blood clotting time compared to the addition of the STARTEM reagent in (1). The addition of the calcium chloride, CTI, kallikrein inhibitor, and FVIIa inhibitor in (3) resulted in the smaller extent of prolongation than that in (2).

This indicates that the tissue factor in the blood of healthy subjects is very few and has only a minor effect on blood coagulation.

The further addition of the human malignant melanoma A375 cell-derived exosome for (2) significantly shortens the blood clotting time, however, the addition of the human malignant melanoma A375 cell-derived exosome for (3) limitedly shortens the blood clotting time. This suggests that the A375-derived exosome contains a large amount of tissue factor.

The shortening of blood clotting time was, on the other hand, limited when the A375-derived exosome was added to (4), (5), and (6).

This suggests that the shortening of blood clotting time by the A375-derived exosome is mainly due to the activation of extrinsic blood coagulation caused by a tissue factor present on a surface of the exosome, and activation of intrinsic blood coagulation due to the acidic phospholipids is considered to be limitedly small.

### Example 8

ROTEM (IL) was used to analyze blood coagulation.

Blood was collected using a blood collection tube (5 mL) containing 3.2% sodium citrate manufactured by Terumo Corporation.

This blood collection tube was sealed with a laminate film, and no rubber stopper is used.

300 µL of whole blood with LPS (final concentration 1 µg/mL) and PMA (final concentration 50 nM) and 300 µL of whole blood without them were each incubated at 37°C for 4 hours followed by addition of the reagents from (1) to (6) in Example 02, respectively, to analyze blood clotting time.

The results of CT and CFT are shown in Table 6.

**[Table 6]**

| No addition of reagents | | | | | | |
|---|---|---|---|---|---|---|
| Control | (1) | (2) | (3) | (4) | (5) | (6) |
| CT | 1, 021 | 2, 388 | 2, 664 | 1, 514 | 1, 737 | 1, 141 |
| CFT | 240 | 391 | - | 309 | 367 | 219 |

| Incubation at 37°C for 4 hours after addition of LPS | | | | | | |
|---|---|---|---|---|---|---|
| LPS | (1) | (2) | (3) | (4) | (5) | (6) |
| CT | 358 | 367 | 961 | 770 | 874 | 828 |
| CFT | 201 | 272 | 291 | 227 | 320 | 247 |

| Incubation at 37°C for 4 hours after addition of PMA | | | | | | |
|---|---|---|---|---|---|---|
| PMA | (1) | (2) | (3) | (4) | (5) | (6) |
| CT | 821 | 1, 557 | 2, 569 | 1, 328 | 1, 375 | 1,231 |
| CFT | 329 | 406 | - | 246 | 393 | 369 |

When the LPS was added to (2), the blood clotting time is greatly shortened. When the LPS was added to (3) as well, the blood clotting time is moderately shortened. Furthermore, the addition of LPS to (4), (5), and (6) as well confirmed shortening of the blood clotting time.

This suggests that the addition of LPS is considered to cause activation of intrinsic blood coagulation in addition to activation of extrinsic blood coagulation by expression of the tissue factor.

The moderate shortening of the blood clotting time occurs, on the other hand, when the PMA was added in (2), however, the shortening of the blood clotting time is limited when the PMA was added in (4), (5) and (6).

These results suggest that the addition of PMA causes weaker activation of extrinsic blood coagulation compared to the case of having added LPS.

### Example 9

ClotPro (Haemonetics Corporation) was used to analyze blood coagulation.

Blood was collected using a blood collection tube (5 mL) containing 3.2% sodium citrate manufactured by Terumo Corporation.

This blood collection tube was sealed with a laminate film, and no rubber stopper is used.

To each of the collected whole blood (340 µL) were added the following reagents of (1) to (6), followed by the addition of any one of reagents from (7) to (12) to analyze blood clotting time.
(1) BP61 (FXII inhibitor), final concentration 5 µg/mL
(2) BP73 (FXII inhibitor) final concentration 5 µg/mL
(3) BP61 (FXII inhibitor) final concentration 5 µg/mL and PKSI-527 (final concentration 40 µM)
(4) BP73 (FXII inhibitor) final concentration 5 µg/mL and PKSI-527 (final concentration 40 µM)
(5) CTI final concentration 20 µg/mL
(6) CTI final concentration 20 µg/mL and PKSI-527 (final concentration 40 µM)
(7) Calcium chloride (final concentration 12 mM)
(8) Calcium chloride (final concentration 12 mM), CTI (final concentration 20 µg/mL), and PKSI-527 (final concentration 40 µM)
(9) Calcium chloride (final concentration 12 mM), BP61 (final concentration 10 µg/mL), and PKSI-527 (final concentration 40 µM)
(10) Calcium chloride (final concentration 12 mM), BP61 (final concentration 20 µg/mL), and PKSI-527 (final concentration 40 µM)
(11) Calcium chloride (final concentration 12 mM), BP73 (final concentration 10 µg/mL), and PKSI-527 (final concentration 40 µM)
(12) Calcium chloride (final concentration 12 mM), BP73 (final concentration 20 µg/mL), and PKSI-527 (final concentration 40 µM)

The results of CT and CFT are shown in Table 7 below.

**[Table 7]**

| | | (7) | (8) | (9) | (10) | (11) | (12) |
|---|---|---|---|---|---|---|---|
| (1) | CT | 1, 507 | 2, 763 | 2, 088 | 2, 893 | 3, 400 | 2, 568 |
| | CFT | 397 | 535 | 712 | - | - | 942 |
| (2) | CT | 1778 | 3082 | 2551 | 2872 | 2689 | 2400 |
| | CFT | 502 | - | 762 | - | 665 | 557 |
| (3) | CT | 3003 | 2455 | 2676 | 3027 | 3272 | - |
| | CFT | 455 | 622 | 832 | - | - | - |
| (4) | CT | 2985 | 2617 | 2793 | 2949 | 2323 | 2984 |
| | CFT | 410 | 305 | 387 | 545 | 967 | 567 |
| (5) | CT | 1239 | 2338 | 2652 | 2535 | 2367 | 2701 |
| | CFT | 317 | 340 | 401 | 452 | 337 | 600 |
| (6) | CT | 1967 | 2763 | 2829 | 2573 | 2835 | 2511 |
| | CFT | 327 | 740 | 500 | 415 | 360 | 422 |

Any of CTI, BP61, and BP73 could be used as the FXII inhibitor. Furthermore, the combination for use with the kallikrein inhibitor inhibited the contact factor and prolonged the blood clotting time.

### Example 10

ClotPro (Haemonetics Corporation) was used to analyze blood coagulation.

Blood was collected using a blood collection tube (5 mL) containing 3.2% sodium citrate manufactured by Terumo Corporation.

This blood collection tube was sealed with a laminated film, and no rubber stopper is used.

To each of whole blood were added the following reagents to analyze coagulation waveforms.
(1) A Startem reagent (IL) (20 µL)
(2) Calcium chloride (final concentration 12 mM), CTI (final concentration 20 µg/mL), and PKSI-527 (final concentration 40 µM)
(3) Calcium chloride (final concentration 12 mM), CTI (final concentration 20 µg/mL), PKSI-527 (final concentration 40 µM), and a TFPI inhibitor (compounds) (final concentration 50 µM)
(4) A Startem reagent (IL) (20 µL) and an A375-derived exosome (final concentration 1 µg/mL)
(5) Calcium chloride (final concentration 12 mM), CTI (final concentration 20 µg/mL), PKSI-527 (final concentration 40 µM), and an A375-derived exosome (final concentration 1 µg/mL)
(6) Calcium chloride (final concentration 12 mM), CTI (final concentration 20 µg/mL), PKSI-527 (final concentration 40 µM), a TFPI inhibitor (compound 3) (final concentration 50 µM), and an A375-derived exosome (final concentration 1 µg/mL)

The results of CT and CFT are shown in Table 8 below.

**[Table 8]**

| | (1) | (2) | (3) | (4) | (5) | (6) |
|---|---|---|---|---|---|---|
| CT | 687 | 2, 921 | 1, 284 | 462 | 496 | 401 |
| CFT | 190 | 477 | 345 | 153 | 150 | 125 |

The addition of A375-derived exosome remarkably enhanced blood coagulation in (5) and (6), allowing evaluation of the hypercoagulation effect.

### Example 11

ROTEM (IL) was used to analyze blood coagulation.

Blood was collected using a blood collection tube (5 mL) containing 3.2% sodium citrate manufactured by Terumo Corporation.

This blood collection tube was sealed with a laminated film, and no rubber stopper is used.

To each of whole blood were added the following reagents to analyze blood coagulation waveforms.
(1) Calcium chloride (final concentration 12 mM), CTI (final concentration 20 µg/mL), PKSI-527 (final concentration 40 µM), an FVIIa inhibitor (A-183X) (final concentration 100 nM), and ecarin (final concentration 1 mU/ml units)
(2) Calcium chloride (final concentration 12 mM), CTI (final concentration 20 µg/mL), PKSI-527 (final concentration 40 µM), an FVIIa inhibitor (A-183X) (final concentration 100 nM), and ecarin (final concentration 2 mU/ml unit)
(3) Calcium chloride (final concentration 12 mM), CTI (final concentration 20 µg/mL), PKSI-527 (final concentration 40 µM), an FVIIa inhibitor (A-183X) (final concentration 100 nM), and ecarin (final concentration 4 mU/ml units)

The results are shown in Table 9 below.

**[Table 9]**

| | (1) | (2) | (3) |
|---|---|---|---|
| CT | 2305 | 1509 | 1234 |
| CFT | 377 | 309 | 404 |

The addition of 1 mU to 4 mU of ecarin as a blood coagulation activator, along with the contact factor inhibitor and the extrinsic blood coagulation inhibitor (FVIIa inhibitor), prolonged blood coagulation to 20 minutes or longer in the blood collected from healthy subjects.

With a normal blood sample (no tissue factor was externally added), if ROTEM waveforms in the case of addition of TFPI and without addition thereof are similar, hypercoagulation in a disease can be predicted to be due to factors other than tissue factors, for example, factors other than extrinsic factors, such as microparticles and platelet activation, and if ROTEM waveforms in the case of addition of TFPI and without addition thereof, cause a clear difference between the two, hypercoagulation in a disease can be predicted to be due to an increase or enhancement of tissue factors.

Furthermore, factors responsible for the blood hypercoagulation can also be analyzed by comparing an evaluation of extrinsic coagulation (addition of the FXII inhibitor and/or the kallikrein inhibitor and the TFPI inhibitor) with an evaluation of intrinsic blood coagulation (including the contact factor inhibitor and the FVII inhibitor).

In such a way, a risk of thrombosis in cancer and the like can be predicted, and its factors can be analyzed.

## Claims

1. A method of evaluating extrinsic blood coagulation ability in vitro, comprising analyzing blood coagulation ability using a blood sample to which a contact factor inhibitor and an inhibitor for a tissue factor pathway inhibitor (TFPI) are added.

2. The method according to claim 1, wherein the contact factor inhibitor is an FXI (blood coagulation factor 11) inhibitor, an FXII (blood coagulation factor 12) inhibitor and/or a kallikrein inhibitor.

3. The method according to claim 1, wherein the contact factor inhibitor is both the FXII inhibitor and the kallikrein inhibitor.

4. The method of analyzing the blood coagulation ability according to any one of claims 1 to 3, wherein the analysis result is compared with that of the blood coagulation ability in the absence of the TFPI inhibitor.

5. A method of evaluating intrinsic blood coagulation ability in vitro, comprising analyzing blood coagulation ability using a blood sample to which both a contact factor inhibitor and an extrinsic blood coagulation inhibitor are added.

6. The method according to claim 5, wherein the extrinsic blood coagulation inhibitor is at least one selected from the group consisting of an FVII (blood coagulation factor 7) inhibitor, a tissue factor inhibitor, and an inactivated FVIIa.

7. The method according to claim 5 or 6, a prothrombin activator and/or a blood coagulation factor 10 activator are further added as blood coagulation activators.

8. The method according to claim 7, wherein the prothrombin activator is ecarin.

9. The method according to claim 7, wherein the blood coagulation factor 10 activator is a Russell's viper venom-derived FX activating enzyme (RVV-FX).

10. The method according to any one of claims 1 to 9, wherein a blood sample is whole blood.

11. A method of analyzing blood coagulation ability, comprising comparing the evaluation of the extrinsic blood coagulation according to any one of claims 1 to 4 with the evaluation of the intrinsic blood coagulation according to any one of claims 5 to 9.

12. The method according to any one of claims 1 to 11, wherein the analysis is conducted by ROTEM (Rotational Thromboelastometry) and/or TEG (Thromboelastography).

13. The method according to any one of claims 1 to 12, wherein the blood sample is a blood sample of a cancer patient or a patient suspected of having cancer.

14. A reagent for analyzing extrinsic blood coagulation ability in vitro, comprising a contact factor inhibitor and a TFPI inhibitor.

15. A reagent for analyzing intrinsic blood coagulation ability in vitro, comprising a contact factor inhibitor and an extrinsic blood coagulation inhibitor.

16. The reagent according to claim 15, further comprising a blood coagulation activator.
